# FASCICULE DE BREVET EUROPEEN

(11) **EP 2 585 503 B1**
(45) Date de publication et mention de la délivrance du brevet: **23.04.2014**
(21) Numéro de dépôt: 11729700.2
(22) Date de dépôt: 08.06.2011
(51) Int. Cl.: C08F 220/06, C08F 220/12, C08F 220/28, C08F 220/58, A61K 8/81, C04B 24/26, C08F 2/22, C08F 283/06, C09D 7/12, C11D 3/37, D21H 21/14

(54) **EMULSIONS ACRYLIQUES ALKALI GONFLABLES A L'ACIDE ACRYLIQUE, LEUR UTILISATION DANS DES FORMULATIONS AQUEUSES ET FORMULATIONS LES CONTENANT**
IN ALKALISCHEN MEDIEN QUELLFÄHIGE ACRYLSÄUREHALTIGE ACRYLEMULSIONEN, IHRE VERWENDUNG IN WÄSSRIGEN FORMULIERUNGEN UND DIESE ENTHALTENDE FORMULIERUNGEN
ALKALI-SWELLABLE ACRYLIC EMULSIONS COMPRISING ACRYLIC ACID, USE THEREOF IN AQUEOUS FORMULATIONS AND FORMULATIONS CONTAINING SAME

(30) Priorité: 25.06.2010 FR 1055077
(43) Date de publication de la demande: 01.05.2013
(73) Titulaire: Coatex S.A.S, 69730 Genay (FR)
(72) Inventeur: SUAU, Jean-Marc, 69480 Lucenay (FR)
(74) Mandataire: Fiorucci, Hélène
(86) Numéro de dépôt international: PCT/IB2011/001285
(87) Numéro de publication internationale: WO 2011/161508

(56) Documents cités:
- FR-A1- 2 360 614
- US-A1- 2007 004 851
- US-A1- 2009 305 934

## Description

La présente invention se rapporte à de nouvelles émulsions épaississantes alkali gonflables, exemptes d'acide méthacrylique : ce monomère était jusqu'alors indispensable dans ce type d'émulsions, ce qui rendait les fabricants dépendants de cette matière première. Ces nouvelles émulsions contiennent une certaine quantité d'acide 2-acrylamido-2-méthylpropane sulfonique (ou AMPS, n° CAS : 40623-75-4). Elles peuvent en outre être fabriquées en l'absence de tensio-actif, ce qui évite la formation de mousse. Enfin, elles s'avèrent efficaces pour épaissir des milieux aqueux, notamment des milieux dont le pH est compris entre 5 et 6, telle que la peau.

On trouve des formulations aqueuses contenant éventuellement des charges minérales dans de très nombreux domaines : celui des peintures aqueuses, des sauces de couchage papetières, des suspensions de matières minérales, des détergents, des formulations cosmétiques, ou encore des bétons et des ciments.

Contrôler leur rhéologie est une nécessité, tant au stade de leur fabrication, que pendant leur transport, leur stockage ou au cours de leur mise en oeuvre. La diversité des contraintes pratiques au niveau de chacune de ces étapes renvoie à une multiplicité de comportements rhéologiques. Dans le cas d'une peinture par exemple, on peut résumer le besoin de l'homme du métier à l'obtention d'un effet d'épaississement de celle-ci, tant pour des raisons de stabilité au cours du temps, que pour une possible application de la peinture sur une surface verticale, l'absence d'éclaboussures au moment de la mise en oeuvre, ou de coulure après la mise en oeuvre, etc...

De fait, on a désigné les produits qui contribuent à cette régulation du comportement rhéologique sous le terme d'épaississants. Parmi ces épaississants, l'homme du métier connaît depuis longtemps la catégorie particulière des émulsions acryliques alkali gonflables, qui sont des polymères mis en émulsion dans l'eau à partir de tensio-actifs, lesdits polymères étant constitués d'au moins un monomère non hydrosoluble et d'au moins un monomère hydrosoluble alkali neutralisable dont l'acide méthacrylique.

La Demanderesse précise que l'expression « émulsion directe d'un polymère dans l'eau » désigne une dispersion stable et homogène de particules de polymère dans l'eau (on ne fait pas référence ici aux émulsions du type huile dans eau ou eau dans huile, qui impliquent l'existence de deux phases distinctes, l'une aqueuse et l'autre huileuse). Quant à l'expression « polymère alkali gonflable », elle signifie que ledit polymère est capable, lorsque le milieu est alcalin, d'incorporer une quantité d'eau telle qu'il y a formation d'un gel.

Il existe 2 grandes familles d'épaississants acryliques alkali gonflables : les épaississants de type ASE (Alkali Swellable Emulsion ou émulsion alcali soluble) et ceux de type HASE (Hydrophobically modified Alkali Swellable Emulsion ou émulsion alcali gonflable et modifiée hydrophobiquement). Les premiers désignent des copolymères de l'acide méthacrylique avec un ester non hydrosoluble de cet acide, et les seconds désignent des copolymères à base d'acide méthacrylique, d'un ester non hydrosoluble de l'acide (méth)acrylique et d'un monomère disposant de groupements hydrophobes dits « associatifs ». Ces copolymères peuvent en outre être réticulés.

Les mécanismes d'action de ces produits diffèrent. Les polymères de type ASE n'épaississent qu'à l'état neutralisé, d'où l'expression « alkali-gonflable » : on induit alors un mécanisme de répulsion ionique entre les différents groupements carboxylates portés par la chaîne polymérique. Ces groupements ionisés polarisent les molécules d'eau ce qui provoque l'augmentation de la viscosité du milieu. En plus du phénomène ionique précité, les polymères de type HASE mettent en jeu des interactions entre leurs groupements hydrophobes associatifs, ce qui contribue aussi à épaissir le milieu. Ces mécanismes, et notamment le caractère alkali gonflable de ces émulsions et leur capacité à épaissir un milieu aqueux à pH voisin de la neutralité, ont été décrits dans les documents WO 2007 / 144721 et « Practical guide to associative thickeners » (Proceedings of the Annual Meeting Technical Program of the FSCT, 2000, 78th, 644-702).

On trouve de nombreuses applications de ces épaississants dans la peinture, les sauces de couchage, ou la cosmétique (voir les demandes de brevet FR 2 693 203 A1, FR 2 872 815 A1, FR 2 633 930 A1, FR 2 872 815 A1). En outre, ils existent sous forme commerciale, notamment à travers les gammes de produits Rheocarb™, Rheocoat™, Thixol™, Rheotech™, Polyphobe™ et Viscoatex™ commercialisées par la société CORTEX S.A.S.

Revenant à la constitution des émulsions de type ASE et HASE, l'acide méthacrylique apparaît aujourd'hui comme un monomère incontournable, qui constitue au moins 20 % en poids de ces polymères épaississants. De part sa fonction carboxylique, il participe à l'augmentation de la viscosité du milieu à travers un phénomène ionique déjà décrit, et il est facilement polymérisable en émulsion aqueuse et en présence de tensio-actifs avec des monomères hydrophobes, dont des monomères associatifs.

Ces polymérisations en émulsion sont largement illustrées dans la littérature scientifique. On pourra se reporter aux publications « Synthesis of an alkali-swellable emulsion and its effect on the rate of polymer diffusion in poly(vinyl acetate-butyl acrylate) latex films » (Journal of Polymer Science, Part A: Polymer Chemistry, 2005, 43 (22), pp. 5632-5642), « Structural and rheological properties of hydrophobically modified alkali-soluble emulsion solutions » (Journal of Polymer Science, Part B: Polymer Physics, 2002, 40 (18), pp. 1985-1994) « Viscoelastic properties of hydrophobically modified alkali-soluble emulsion in salt solutions » (Polymer, 1999, 40 (23), pp. 6369-6379), « Dissolution behavior in water of a model hydrophobic alkali-swellable emulsion polymer with C20H41 groups « (Canadian Journal of Chemistry (1998), 76(11), pp. 1779-1787), et aux demandes de brevet EP 0 089 213 A1, EP 0 646 606 A1, EP 0 979 833 A1, pour les ASE et EP 0 013 836 A1, WO 93 / 2454 A1, US 4268 641 A1, US 4 421 902 A1, US 3 915 921 A1 pour les HASE.

Aujourd'hui, l'acide méthacrylique est considéré comme un monomère indispensable dans les émulsions de type ASE et HASE : cette donnée est largement intégrée dans le monde industriel et n'est pas remise en cause par l'homme du métier. Mais c'est précisément le caractère obligatoire de l'acide méthacrylique qui pose un problème. Nous vivons en effet une époque où la pénurie des matières premières et la fluctuation de leur coût indexée sur celui du baril de pétrole brut constituent une donnée récurrente dans l'industrie chimique ; par conséquent, une technologie annexée à une matière première exclusive, devient stratégiquement risquée pour ses fabricants et ses utilisateurs.

C'est pour remédier à cet inconvénient majeur que la Demanderesse a entrepris des recherches en vue de substituer l'acide acrylique à l'acide méthacrylique dans les émulsions de type ASE et HASE. Or, aucun document de l'état de la technique ne divulgue une technique de polymérisation de l'acide acrylique en émulsion directe, sans acide méthacrylique et en présence d'un monomère hydrophobe, l'acide acrylique représentant alors au moins 20 % en poids des monomères à polymériser.

La Demanderesse est parvenue à démontrer qu'une telle polymérisation en émulsion directe était possible, dans la mesure où on mettait en oeuvre une certaine quantité d'un monomère particulier qui est l'acide 2-acrylamido-2-méthylpropane sulfonique (ou AMPS, n° CAS : 40623-75-4). De manière tout à fait surprenante et avantageuse, on parvient ainsi à fabriquer de véritables émulsions de type ASE et HASE exemptes d'acide méthacrylique.

Or, les épaississants à base d'acide acrylique connus de l'homme du métier sont des polymères de l'acide acrylique réticulés obtenus par précipitation dans un solvant (voir le document EP 0 584 771 A1), ou des polymères réticulés ou modifiés hydrophobiquement en émulsion inverses eau dans huile (voir les documents FR 2 873 126 A1 et FR 2 782 086 A1), ou enfin des polymères de hauts poids moléculaires (supérieurs à 10 000 000 g/mol) obtenus en solution dans l'eau et séchés (connus sous le nom de polymères superabsorbants ou SAP ; voir notamment les documents EP 0 371 421 A1 et US 5 221 722 A1). Rien n'existe dans l'état de la technique, en vue de réaliser des émulsions alkali gonflables, contenant aux moins 20 % en poids d'acide acrylique et exemptes d'acide méthacrylique.

De plus, le procédé objet de la présente Demande, dispose d'une variante préférentielle qui apporte d'autres avantages : il peut être mise en oeuvre sans tensio-actif ni solvant autre que l'eau. Or, il est bien connu que ces derniers génèrent de nombreux problèmes dans la mise en oeuvre des émulsions de type ASE et HASE, au sein d'une formulation aqueuse.

Dans le cas d'une peinture, il peut y avoir création d'inhomogénéités, et même altération des propriétés du produit final c'est-à-dire du film sec de peinture résultant du séchage de la formulation aqueuse. On peut aussi observer la formation de « cratères » ou de particules insolubles qui sont autant d'hétérogénéités nuisant à l'aspect esthétique et aux propriétés de surface du film (aspect mécanique, mais aussi propriétés optiques et état de surface). Enfin, il est bien connu que la présence de tensio-actifs dans une formulation de peinture va dégrader au final le caractère lessivable du film sec (voir « Effect of surfactants used for binder synthesis on the properties of latex paints », Progress in Organic Coatings, 2005, 53 (2), pp. 112-118).

D'autres avantages des nouvelles émulsions de type ASE et HASE sans acide méthacrylique résident dans le fait qu'elles présentent un extrait sec commercial (supérieur à 25 % en poids sec de matière active), sont stables dans le temps et caractérisées par des tailles de particules similaires aux émulsions de l'art antérieur ; enfin, leur efficacité épaississante est démontrée.

Un autre objet de la présente invention réside en l'utilisation des émulsions précitées comme agent épaississant dans des formulations aqueuses, celles-ci constituant le dernier objet de la présente invention.

Aussi, un premier objet de la présente invention consiste en un procédé de fabrication en émulsion aqueuse directe d'un polymère, caractérisé en ce que ledit procédé met en jeu la réaction de polymérisation de, exprimé en % en poids de chacun des monomères :
a) 20 % à 80 % en poids d'acide acrylique,
b) 10 % à 90 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
   le total a) + b) + c) + d) étant égal à 100 %,
ou
a) 20 % à 80 % en poids d'acide acrylique,
b) 10 % à 90 % en poids d'au moins un ester de l'acide (méth)acrylique,
c) 10 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
   le total a) + b) + c) + d) + e) étant égal à 100 %,

Les autres caractéristiques de ce procédé (température, choix du système catalytique, mise en oeuvre d'un agent de transfert, emploi d'un éventuel réticulant) sont celles décrites dans l'état de la technique, notamment à travers les documents précités auxquels l'homme du métier pourra se référer.

Ce procédé est aussi caractérisé en ce que l'ester de l'acide (méth)acrylique est choisi parmi l'acrylate d'éthyle, l'acrylate de butyle, le méthacrylate de méthyle et leurs mélanges.

Ce procédé est aussi caractérisé en ce que le monomère contenant un groupement hydrophobe possède la formule générale : 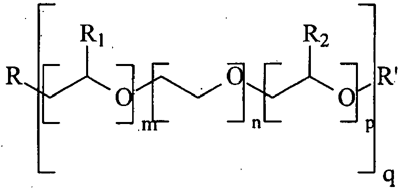 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

Ce procédé est aussi caractérisé en ce que le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

Ce procédé est aussi caractérisé en ce que l'émulsion aqueuse présente un extrait sec compris entre 10 % et 50 % en poids sec de polymère, par rapport à son poids total.

Ce procédé est aussi caractérisé en ce que l'émulsion présente une taille de particules comprise entre 50 nm et 500 nm.

Ce procédé est aussi caractérisé en ce que le polymère présente une masse molaire moyenne en poids comprise entre 20 000 g/mol et 1 000 000 g/mol.

Dans une variante préférée, ce procédé ne met pas en oeuvre de tensio-actif ni de solvant autre que l'eau.

Un autre objet de la présente invention consiste en l'utilisation, dans un procédé de fabrication d'une émulsion de type ASE ou HASE, d'acide 2-acrylamido-2-méthylpropane sulfonique, comme agent permettant de substituer complètement l'acide méthacrylique par l'acide acrylique.

Un autre objet de la présente invention consiste en une émulsion aqueuse directe, d'un polymère caractérisé en ce qu'il est constitué de, exprimé en % en poids de chacun des monomères :
a) 20 % à 80 % en poids d'acide acrylique,
b) 10 % à 90 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
   le total a) + b) + c) + d) étant égal à 100 %,
ou
a) 20 % à 80 % en poids d'acide acrylique,
b) 10 % à 90 % en poids d'au moins un ester de l'acide (méth)acrylique,
c) 10 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
   le total a) + b) + c) + d) + e) étant égal à 100 %,

Cette émulsion est aussi caractérisée en ce que le monomère contenant un groupement hydrophobe possède la formule générale : 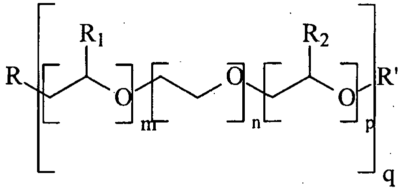 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

Cette émulsion est aussi caractérisée en ce que le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

Cette émulsion est aussi caractérisée en ce qu'elle présente un extrait sec compris entre 10 % et 50 % en poids sec de polymère, par rapport à son poids total.

Cette émulsion est aussi caractérisée en ce qu'elle présente une taille de particules comprise entre 50 nm et 500 nm.

Cette émulsion est aussi caractérisée en ce que le polymère présente une masse molaire moyenne en poids comprise entre 20 000 g/mol et 1 000 000 g/mol.

Dans une variante préférée, cette émulsion est caractérisée en ce qu'elle est exempte de tensio-actifs et de solvant autre que l'eau.

Un autre objet de la présente invention réside dans l'utilisation de l'émulsion précitée, comme agent épaississant d'une formulation aqueuse. Concrètement, l'émulsion est introduite dans le milieu à épaissir, dont on règle le pH au voisinage de la neutralité, afin d'obtenir l'effet d'épaississement. L'homme du métier, à partir d'expériences de routine, saura trouver le pH à partir duquel on observe le phénomène d'épaississement.

Cette utilisation est aussi caractérisée en ce que ladite formulation est choisie parmi une peinture aqueuse, une sauce de couchage papetière, une suspension aqueuse de matières minérales, un détergent, une formulation cosmétique, ou contenant un liant hydraulique, et enfin une formulation cosmétique, plus particulièrement une formulation cosmétique destinée à être appliquée sur la peau ou sur une région du corps dont le pH est compris entre 5 et 6.

Un dernier objet de la présente invention réside dans une formulation aqueuse contenant l'émulsion précitée, ladite formulation étant choisie parmi une peinture aqueuse, une sauce de couchage papetière, une suspension aqueuse de matières minérales, un détergent, une formulation cosmétique, ou contenant un liant hydraulique, une formulation cosmétique destinée à être appliquée sur la peau ou sur une région du corps dont le pH est compris entre 5 et 6.

### EXEMPLES

### Exemple 1

### Essai n° 1 selon l'invention

Cet essai concerne la fabrication d'une émulsion de type ASE, contenant un copolymère constitué de, exprimé en % en poids de chacun de ses monomères :
a) 36,4 % d'acide acrylique,
b) 62,4 % d'acrylate d'éthyle,
c) 1,2 % d'AMPS

Dans un réacteur de 1 litre muni d'une agitation mécanique et d'un système de chauffage de type bain d'huile, on introduit 550 g d'eau bipermutée et 7,0 g d'une solution d'AMPS (solution à 55 % dans l'eau d'AMPS neutralisé par de la soude, commercialisé par la société LUBRIZOL™ sous le nom AMPS 2405).

On chauffe le milieu jusqu'au 78°C puis on introduit par un entonnoir le système catalytique constitué de 0,96 g de persulfate d'ammonium dissous dans 10 g d'eau bipermutée et 0,096 g de métabisulfite de sodium dissous dans 10 g d'eau bipermutée.

On introduit ensuite, de manière continue et progressive 236 g d'acrylate d'éthyle et 85 g d'acide acrylique (à 90%).
Pendant toute la durée de l'ajout, la température du milieu réactionnel est maintenue à 78°C (±2).

Une fois l'ajout terminé, on rince la pompe avec 20 g d'eau bipermutée puis on laisse réagir pendant 30 minutes à 78°C (±2).

On ajoute alors 0,10 g de persulfate d'ammonium dissous dans 25 g d'eau en 30 minutes tout en maintenant la température à 80°C (±2) et on laisse réagir pendant 1 heure à 87°C (±2).

On obtient alors une émulsion contenant 32,4 % en poids sec de matière active, et dont la taille des particules, mesurée par Diffusion Dynamique de la Lumière est égale à 99 nm.

### Essais comparatifs

On a tout d'abord cherché à fabriquer le même polymère que dans l'essai 1, mais sans mettre en oeuvre d'AMPS, à savoir un polymère constitué de 36,5 % en poids d'acide acrylique et 63,5 % en poids d'acrylate d'éthyle.

Pour ce faire, on a mis en oeuvre le même protocole que précédemment, exception faite de l'introduction initiale d'AMPS. Après avoir laissé réagir à 87°C pendant 1 heure, on observe la formation d'un précipité trouble en solution, dont une partie se fixe au niveau de l'axe du réacteur (enrochement). La taille des particules formées est supérieure 500 nm. Le milieu, très inhomogène et riche en grosses particules, ne se prête pas à des manipulations, et notamment à des opérations de pompage. Le stockage risque de plus de conduire à la sédimentation du produit.

On a ensuite mis en oeuvre une quantité d'AMPS supérieure à 22 % en poids de la masse totale des monomères engagés, selon un mode opératoire indentique à celui décrit dans l'essai n° 1, le copolymère étant alors constitué, exprimé en % en poids de chacun de ses monomères :
a) 36,5 % d'acide acrylique,
b) 38,5 % d'acrylate d'éthyle,
c) 25,0 % d'AMPS.

On observe alors la formation d'espèces insolubles qui précipitent dans le milieu.

On a ensuite remplacé l'AMPS (1,2 %, poids pour poids) par un autre monomère mis en oeuvre dans les émulsions ASE et HASE : l'acrylate d'éthyle. On ne parvient pas à réaliser des émulsions homogènes ; les observations sont les mêmes que précédemment, avec la formation d'un précipité, le phénomène d'enrochement, et les conséquences néfastes qui s'en suivent.

On a ensuite substitué l'AMPS (toujours 1,2 % poids pour poids) par du styrène, du méthacrylate de lauryle, du méthacrylate de 2-sulfoéthyle, du styrène sulfonate de sodium, du sel de sodium du 1-allyloxy-2-hydroxypropyl sulfonate (Sipomer COPS 1) pour des résultats identiques (présence d'insolubles, et phénomène d'enrochement).

### Essai n° 2 à 11 selon l'invention

Les essais n° 2 à 11 concernent la synthèse d'autres émulsions illustrant l'invention, selon le même mode opératoire que décrit précédemment.
Les essais n° 2 à 8 illustrent d'autres compositions monomériques avec un taux massique d'AMPS fixé à 1,2 %, alors que les essais n° 9 à 11 illustrent d'autres taux en AMPS (on a alors maintenu le constant le ratio massique entre l'acrylate d'éthyle et l'acide acrylique).

Les caractéristiques des émulsions obtenues sont données dans le tableau 1.

**Tableau 1**

| **Essai n°** | **Composition monomérique (% massique)** | **ES (%)** | **Diamètre particule (nm)** |
|---|---|---|---|
| 1 | 1,2 AMPS / 62,4 AE / 36,4 AA | 32,4 | 99 |
| 2 | 1,2 AMPS / 68,7 AE / 30,1 AA | 32,8 | 115 |
| 3 | 1,2 AMPS / 74,6 AE / 24,2 AA | 29,6 | 153 |
| 4 | 1,2 AMPS / 80,4 AE / 18,4 AA | 32,2 | 102 |
| 5 | 1,2 AMPS / 28,35 ABu / 70,45 AA | 37,6 | 287 |
| 6 | 1,2 AMPS / 74,1 AE / 18,4 AA / 6,3 MAM | 33,2 | 96 |
| 7 | 1,2 AMPS / 67,8 AE / 18,4 AA / 12,6 MAM | 32,4 | 106 |
| 8 | 1,2 AMPS / 53,25 AE / 35,8 AA / 9,75 MethC22OE25 | 33,1 | 185 |
| 9 | 10 AMPS / 57,9 AE / 32,1 AA | 29,6 | 175 |
| 10 | 15 AMPS / 55,4 AE / 29,6 AA | 29,6 | 215 |
| 11 | 20 AMPS / 52,9 AE / 27,1 AA | 29,6 | 276 |

Dans ce tableau, MethC22OE2 désigne un monomère de formule (I) dans laquelle R désigne la fonction méthacrylate, m=p=0, n=25, et R' désigne un groupe méthyle, AE désigne l'acrylate d'éthyle, ABu l'acrylate de butyle, AMA le méthacrylate de méthyle, AA et AMA les acides acrylique et méthacrylique.

### Exemple 2

Cet exemple illustre le pouvoir épaississant des émulsions selon l'invention, mises en oeuvre dans de l'eau de manière à avoir une teneur en matière active de 5 % en polymère sec.
Après introduction dans l'eau, le milieu est neutralisé par ajout de soude à un pH compris entre 4,9 et 5,5 et on mesure la viscosité Brookfield™ du milieu, à 25 °C et à 100 tours / minute, dont les valeurs sont reportées dans le tableau 2

**Tableau 2**

| **Emulsion selon l'essai n°** | **Viscosité Brookfield™ (mPa.s) du gel à 5 %** |
|---|---|
| 1 | 3320 |
| 9 | 2830 |
| 10 | 1750 |
| 11 | 1310 |

On démontre ainsi le caractère épaississant des émulsions fabriquées, dès l'instant où elles sont placées dans des conditions alcalines.

Par ailleurs, il est surprenant de noter que l'effet épaississant se manifeste pour un pH aussi bas ; habituellement, les émulsions de type ASE et HASE épaississent dans une zone de pH comprise entre 6 et 7.

Cet épaississement à « bas pH » est particulièrement difficile à obtenir, et est tout à fait intéressant dans le domaine de la cosmétique, où on recherche un effet épaississant à un pH voisin de la peau, c'est-à-dire dans une zone comprise entre 5 et 6 unités pH. Ceci est rapporté dans le document EP 2 027 169 A1.

### Exemple 3

Cet exemple illustre la mise en oeuvre, dans la formulation d'un béton, d'émulsions épaississantes de type ASE commerciales (contenant des tensio-actifs) et d'une émulsion selon l'invention exempte de tensio-actif.

Pour ce faire, on réalise un béton selon les techniques bien décrites dans la littérature, constitué de :
- 300 kg de ciment CEM 1 52.5 N ;
- 880 de gros cailloux 10/20 ;
- 110 kg de sable 0/4 ;
   dont le rapport eau sur ciment E / C est réglé à 0,5, et dans lequel on introduit, par rapport au poids sec de ciment :
- 1,23 % en poids en l'état d'un dispersant commercialisé par la société COATEX™, sous le nom Ethacryl™ 1030 ;
- 1 % en poids en l'état d'un antimousse commercialisé par la société HUNTSMANN™ sous le nom Empilan PF 7169.

Les essais A, B, C et D, mettent respectivement en oeuvre dans la formulation de béton 0,7 % en poids en l'état :
- d'une émulsion de type ASE contenant des tensio-actifs et commercialisée par la société COATEX™ sous le nom Viscoatex™ 730 ;
- d'une émulsion de type ASE contenant des tensio-actifs et commercialisée par la société COATEX™ sous le nom Rheocoat™ 35 ;
- d'une émulsion de type HASE contenant des tensio-actifs et commercialisée par la société COATEX™ sous le nom Rheocoat™ 66 ;
- d'une émulsion selon l'invention, qui est celle décrite dans l'essai n° 1.

Pour chacun des essais A, B, C et D, on a mesuré l'air occlus, selon la norme EN 12350-7. On obtient respectivement 9,5 %, 12,0 %, 12,5 % et 3,0 %.

L'émulsion épaississante selon l'invention permet donc de diminuer très sensiblement la quantité d'air introduite dans la formulation. On dispose donc au final d'un produit plus compact, à la résistance améliorée.

## Revendications

1. Procédé de fabrication en émulsion aqueuse directe d'un polymère, **caractérisé en ce que** ledit procédé met en jeu la réaction de polymérisation de, exprimé en % en poids de chacun des monomères :
a) 20 % à 80 % en poids d'acide acrylique,
b) 10 % à 90 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %,
ou
a) 20 % à 80 % en poids d'acide acrylique,
b) 10 % à 90 % en poids d'au moins un ester de l'acide (méth)acrylique,
c) 10 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
et **en ce que** l'émulsion aqueuse présente un extrait sec compris entre 10 % et 50 % en poids sec de polymère, par rapport à son poids total.

2. Procédé selon la revendication 1, **caractérisé en ce que** l'ester de l'acide (méth)acrylique est choisi parmi l'acrylate d'éthyle, l'acrylate de butyle, le méthacrylate de méthyle et leurs mélanges.

3. Procédé selon une des revendications 1 ou 2, **caractérisé en ce que** le monomère contenant un groupement hydrophobe possède la formule générale : 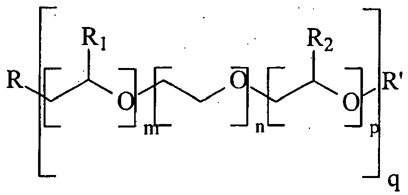 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

4. Procédé selon une des revendications 1 à 3, **caractérisé en ce que** le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

5. Procédé selon une des revendications 1 à 4, **caractérisé en ce que** l'émulsion présente une taille de particules comprise entre 50 nm et 500 nm.

6. Procédé selon une des revendications 1 à 5, **caractérisé en ce que** le polymère présente une masse molaire moyenne en poids comprise entre 20 000 g/mol et 1 000 000 g/mol.

7. Procédé selon une des revendications 1 à 6, **caractérisé en ce qu'**il ne met pas en oeuvre de tensio-actif ni de solvant autre que l'eau.

8. Emulsion aqueuse directe, d'un polymère **caractérisé en ce qu'**il est constitué de, exprimé en % en poids de chacun des monomères :
a) 20 % à 80 % en poids d'acide acrylique,
b) 10 % à 90 % en poids d'au moins un ester de l'acide (méth)acrylique,
c) 0,05 % à 22 % en poids d'acide 2-acrylamido-2-méthylpropane sulfonique,
d) 0 à 1 % en poids d'au moins un monomère réticulant,
le total a) + b) + c) + d) étant égal à 100 %,
ou
a) 20 % à 80 % en poids d'acide acrylique,
b) 10 % à 90 % en poids d'au moins un ester de l'acide (méth)acrylique,
c) 10 % à 25 % en poids d'un monomère contenant un groupement hydrophobe,
d) 0,05 % à 22 % en poids d'acide 2-acrylamido-2-méthylpropane sulfonique,
e) 0 à 1 % en poids d'au moins un monomère réticulant,
le total a) + b) + c) + d) + e) étant égal à 100 %,
et **en ce que** l'émulsion aqueuse présente un extrait sec compris entre 10 % et 50 % en poids sec de polymère, par rapport à son poids total

9. Emulsion selon la revendication 8, **caractérisée en ce que** le monomère contenant un groupement hydrophobe possède la formule générale : 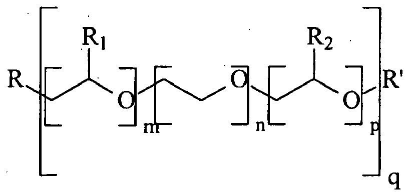 où :
- m, n, p et q sont des entiers et m, n, p sont inférieurs à 150, q est supérieur à 0 et au moins un entier parmi m, n et p est non nul,
- R comporte une fonction vinylique polymérisable,
- R₁ et R₂ sont identiques ou différents et représentent des atomes d'hydrogène ou des groupements alkyls,
- R' est un groupement hydrophobe comportant au moins 6 et au plus 36 atomes de carbone.

10. Emulsion selon une des revendications 8 ou 9, **caractérisée en ce que** le monomère réticulant est choisi parmi le diméthacrylate d'éthylène glycol, le triméthylolpropanetriacrylate, le diallyl phthalate, l'acrylate d'allyle, les maléates d'allyle, le méthylène-bis-acrylamide, le méthylène-bis-méthacrylamide, le tétrallyloxyéthane, les triallylcyanurates, les éthers allyliques obtenus à partir de polyols.

11. Emulsion selon une des revendications 8 à 10, **caractérisée en ce qu'**elle présente une taille de particules comprise entre 50 nm et 500 nm.

12. Emulsion selon une des revendications 8 à 11, **caractérisée en ce que** le polymère présente une masse molaire moyenne en poids comprise entre 20 000 g/mol et 1 000 000 g/mol.

13. Emulsion selon une des revendications 8 à 12, **caractérisée en ce qu'**elle est exempte de tensio-actifs et de solvant autre que l'eau.

14. Utilisation de l'émulsion selon une des revendications 8 à 13, comme agent épaississant d'une formulation aqueuse.

15. Utilisation selon la revendication 14, **caractérisée en ce que** ladite formulation est choisie parmi une peinture aqueuse, une sauce de couchage papetière, une suspension aqueuse de matières minérales, un détergent, une formulation cosmétique, ou contenant un liant hydraulique, plus particulièrement une formulation cosmétique destinée à être appliquée sur la peau ou sur une région du corps dont le pH est compris entre 5 et 6.

16. Formulation aqueuse contenant l'émulsion selon une des revendications 8 à 12, ladite formulation étant choisie parmi une peinture aqueuse, une sauce de couchage papetière, une suspension aqueuse de matières minérales, un détergent, une formulation cosmétique, ou contenant un liant hydraulique, une formulation cosmétique destinée à être appliquée sur la peau ou sur une région du corps dont le pH est compris entre 5 et 6.

## Patentansprüche

1. Verfahren zur Herstellung eines Polymers in direkter wässriger Emulsion, **dadurch gekennzeichnet, dass** bei dem Verfahren die folgenden Stoffe eine Polymerisationsreaktion erfahren, ausgedrückt in Gewichts-% jedes der Monomere:
a) 20 bis 80 Gewichts-% an Acrylsäure,
b) 10 bis 90 Gewichts-% mindestens eines (Meth)acrylsäureesters,
c) 0,05 bis 22 Gewichts-% an 2-Acrylamido-2-methylpropansulfonsäure,
d) 0 bis 1 Gewichts-% mindestens eines vernetzenden Monomers,
wobei a) +b) + c) + d) zusammengenommen gleich 100% sind,
oder
a) 20 bis 80 Gewichts-% an Acrylsäure,
b) 10 bis 90 Gewichts-% mindestens eines (Meth)acrylsäureesters,
c) 10 bis 25 Gewichts-% eines Monomers, welches eine hydrophobe Gruppe enthält,
d) 0,05 bis 22 Gewichts-% an 2-Acrylamido-2-methylpropansulfonsäure,
e) 0 bis 1 Gewichts-% mindestens eines vernetzenden Monomers,
wobei a) +b) + c) + d) + e) zusammengenommen gleich 100% sind,
dadurch, dass die wässrige Emulsion einen Trockenextrakt im Bereich von 10 % bis 50 % nach Trockengewicht an Polymer aufweist, bezogen auf ihr Gesamtgewicht.

2. Verfahren nach Anspruch 1, **dadurch gekennzeichnet, dass** der (Meth)acrylsäureester aus Ethylacrylat, Butylacrylat, Methylmethacrylat und deren Mischungen ausgewählt ist.

3. Verfahren nach einem der Ansprüche 1 oder 2, **dadurch gekennzeichnet, dass** das Monomer, welches eine hydrophobe Gruppe enthält, die folgende allgemeine Formel besitzt: 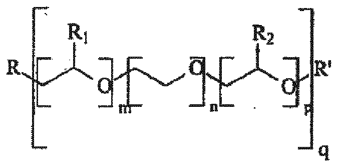 wobei:
- m, n, p und q ganze Zahlen sind und m, n, p kleiner als 150 sind, q größer als 0 ist, und mindestens eine der ganzen Zahlen m, n und p ungleich Null ist,
- R eine polymerisierbare funktionelle Vinylgruppe aufweist,
- R₁ und R₂ identisch oder verschiedenartig sind und Wasserstoffatome oder Alkylgruppen darstellen,
- R' eine hydrophobe Gruppe ist, die mindestens 6 und höchstens 36 Kohlenstoffatome aufweist.

4. Verfahren nach einem der Ansprüche 1 bis 3, **dadurch gekennzeichnet, dass** das vernetzende Monomer aus Ethylenglycoldimethacrylat, Trimethylolpropantriacrylat, Diallylacrylat, Allylacrylat, den Allylmaleaten, Methylen-bis-acrylamid, Methylen-bis-methacrylamid, Tetrallyloxyethan, den Triallylcyanuraten, den Allylethern, welche ausgehend von Polyolen erhalten werden, ausgewählt ist.

5. Verfahren nach einem der Ansprüche 1 bis 4, **dadurch gekennzeichnet, dass** die Emulsion eine Partikelgröße im Bereich von 50 bis 500 nm aufweist.

6. Verfahren nach einem der Ansprüche 1 bis 5, **dadurch gekennzeichnet, dass** das Polymer ein Gewichtsmittel des Molekulargewichts im Bereich von 20.000 g/mol bis 1.000.000 g/mol aufweist.

7. Verfahren nach einem der Ansprüche 1 bis 6, **dadurch gekennzeichnet, dass** dabei weder ein Tensid noch ein Lösemittel außer Wasser zum Einsatz kommt.

8. Direkte wässrige Emulsion eines Polymers, welches **dadurch gekennzeichnet ist, dass** es aus Folgendem besteht, ausgedrückt in Gewichts-% jedes der Monomere:
a) 20 bis 80 Gewichts-% an Acrylsäure,
b) 10 bis 90 Gewichts-% mindestens eines (Meth)acrylsäureesters,
c) 0,05 bis 22 Gewichts-% an 2-Acrylamido-2-methylpropansulfonsäure,
d) 0 bis 1 Gewichts-% mindestens eines vernetzenden Monomers,
wobei a) +b) + c) + d) zusammengenommen gleich 100% sind,
oder
a) 20 bis 80 Gewichts-% an Acrylsäure,
b) 10 bis 90 Gewichts-% mindestens eines (Meth)acrylsäureesters,
c) 10 bis 25 Gewichts-% eines Monomers, welches eine hydrophobe Gruppe enthält,
d) 0,05 bis 22 Gewichts-% an 2-Acrylamido-2-methylpropansulfonsäure,
e) 0 bis 1 Gewichts-% mindestens eines vernetzenden Monomers,
wobei a) +b) + c) + d) + e) zusammengenommen gleich 100% sind,
und dadurch, dass die wässrige Emulsion einen Trockenextrakt im Bereich von 10 % bis 50 % nach Trockengewicht an Polymer aufweist, bezogen auf ihr Gesamtgewicht.

9. Emulsion nach Anspruch 8, **dadurch gekennzeichnet, dass** das Monomer, welches eine hydrophobe Gruppe enthält, die folgende allgemeine Formel besitzt: 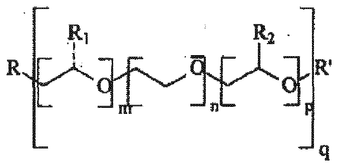 wobei:
- m, n, p und q ganze Zahlen sind und m, n, p kleiner als 150 sind, q größer als 0 ist, und mindestens eine der ganzen Zahlen m, n und p ungleich Null ist,
- R eine polymerisierbare funktionelle Vinylgruppe aufweist,
- R₁ und R₂ identisch oder verschiedenartig sind und Wasserstoffatome oder Alkylgruppen darstellen,
- R' eine hydrophobe Gruppe ist, die mindestens 6 und höchstens 36 Kohlenstoffatome aufweist.

10. Emulsion nach einem der Ansprüche 8 oder 9, **dadurch gekennzeichnet, dass** das vernetzende Monomer aus Ethylenglycoldimethacrylat, Trimethylolpropantriacrylat, Diallylacrylat, Allylacrylat, den Allylmaleaten, Methylen-bis-acrylamid, Methylen-bismethacrylamid, Tetrallyloxyethan, den Triallylcyanuraten, den Allylethern, welche ausgehend von Polyolen erhalten werden, ausgewählt ist.

11. Emulsion nach einem der Ansprüche 8 bis 10, **dadurch gekennzeichnet, dass** sie eine Partikelgröße im Bereich von 50 nm bis 500 nm aufweist.

12. Emulsion nach einem der Ansprüche 8 bis 11, **dadurch gekennzeichnet, dass** das Polymer ein Gewichtsmittel des Molekulargewichts im Bereich von 20.000 g/mol bis 1.000.000 g/mol aufweist.

13. Emulsion nach einem der Ansprüche 8 bis 12, **dadurch gekennzeichnet, dass** sie frei von Tensiden und von Lösemittel außer Wasser ist.

14. Verwendung der Emulsion nach einem der Ansprüche 8 bis 13 als Verdickungsmittel für eine wässrige Formulierung.

15. Verwendung nach Anspruch 14, **dadurch gekennzeichnet, dass** die Formulierung aus einem wässrigen Anstrichmittel, einer Papierstreichmasse, einer wässrigen Suspension mineralischer Stoffe, einem Reinigungsmittel, einer Formulierung, die kosmetischen Zwecken dient oder ein hydraulisches Bindemittel enthält, ausgewählt ist, insbesondere aus einer kosmetischen Formulierung, die dafür bestimmt ist, auf die Haut oder auf eine Körperregion, deren pH-Wert im Bereich von 5 bis 6 liegt, aufgebracht zu werden.

16. Wässrige Formulierung, welche die Emulsion nach einem der Ansprüche 8 bis 12 enthält, wobei die Formulierung aus einem wässrigen Anstrichmittel, einer Papierstreichmasse, einer wässrigen Suspension mineralischer Stoffe, einem Reinigungsmittel, einer Formulierung, die kosmetischen Zwecken dient oder ein hydraulisches Bindemittel enthält, einer kosmetischen Formulierung, die dafür bestimmt ist, auf die Haut oder auf eine Körperregion, deren pH-Wert im Bereich von 5 bis 6 liegt, aufgebracht zu werden, ausgewählt ist.

## Claims

1. Direct manufacturing process in an aqueous emulsion of a polymer, **characterized in that** said process involves the polymerization reaction of, expressed in % by weight of each of the monomers:
a) 20% to 80% by weight of acrylic acid,
b) 10% to 90% by weight of at least one ester of (meth)acrylic acid,
c) 0.05% to 22% by weight of 2-acrylamido-2-methylpropane sulfonic acid,
d) 0 to 1% by weight of at least one crosslinking monomer,
the total a) + b) + c) + d) being equal to 100%,
or
a) 20% to 80% by weight of acrylic acid,
b) 10% to 90% by weight of at least one ester of (meth)acrylic acid,
c) 10% to 25% by weight of a monomer containing a hydrophobic group,
d) 0.05% to 22% by weight of 2-acrylamido-2-methylpropane sulfonic acid,
e) 0 to 1% by weight of at least one crosslinking monomer,
the total a) + b) + c) + d) +e) being equal to 100%,
and **in that** the aqueous emulsion presents a dry extract that is between 10% and 50% by dry weight of polymer with respect to its total weight.

2. Process according to claim 1, **characterized in that** the ester of (meth)acrylic acid is chosen from ethyl acrylate, butyl acrylate, methyl methacrylate, and their mixtures.

3. Process according to claims 1 or 2, **characterized in that** the monomer containing a hydrophobic group has the general formula: 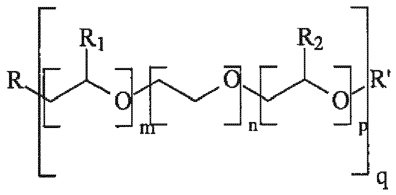 where:
m, n, p and q are integers and m, n, p are less than 150, q is greater than 0 and at least one integer from among m, n and p is non-zero,
- R contains a polymerizable vinyl function,
- R₁ and R₂ are identical or different and represent hydrogen atoms or alkyl groups,
- R' is a hydrophobic group with at least 6 and not more than 36 carbon atoms.

4. Process according to one of claims 1 to 3, **characterized in that** the crosslinking monomer is chosen from among ethylene glycol dimethacrylate, trimethylolpropane triacrylate, diallyl phthalate, allyl acrylate, the allyl maleates, methylene-bis-acrylamide, methylene-bis-methacrylamide, tetrallyloxyethane, the triallyl cyanurates, the allylic ethers obtained from polyols.

5. Process according to one of claims 1 to 4, **characterized in that** the emulsion has a particle size between 50 nm and 500 nm.

6. Process according to one of claims 1 to 5, **characterized in that** the polymer presents a mean molar mass by weight of between 20,000 g/mol and 1,000,000 g/mol.

7. Process according to one of claims 1 to 6, **characterized in that** it does not use a surfactant or solvent other than water.

8. Direct aqueous emulsion of a polymer **characterized in that** it consists of, expressed in % by weight of each monomer:
a) 20% to 80% by weight of acrylic acid,
b) 10% to 90% by weight of at least one ester of (meth)acrylic acid,
c) 0.05% to 22% by weight of 2-acrylamido-2-methylpropane sulfonic acid,
d) 0 to 1% by weight of at least one crosslinking monomer,
the total a) + b) + c) + d) being equal to 100%,
or
a) 20% to 80% by weight of acrylic acid,
b) 10% to 90% by weight of at least one ester of (meth)acrylic acid,
c) 10% to 25% by weight of a monomer containing a hydrophobic group,
d) 0.05% to 22% by weight of 2-acrylamido-2-methylpropane sulfonic acid,
e) 0 to 1% by weight of at least one crosslinking monomer,
the total a) + b) + c) + d) + e) being equal to 100%,
and **in that** the aqueous emulsion presents a dry extract that is between 10% and 50% by dry weight of polymer with respect to its total weight.

9. Emulsion according to claim 8, **characterized in that** the monomer containing a hydrophobic group has the general formula: 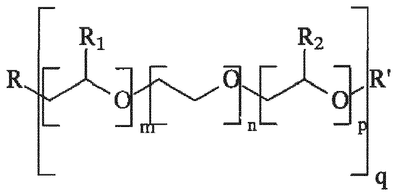 where:
- m, n, p and q are integers and m, n, p are less than 150, q is greater than 0 and at least one integer from among m, n and p is non-zero,
- R contains a polymerizable vinyl function,
- R₁ and R₂ are identical or different and represent hydrogen atoms or alkyl groups,
- R' is a hydrophobic group with at least 6 and not more than 36 carbon atoms.

10. Emulsion according to one of claims 8 or 9, **characterized in that** the crosslinking monomer is chosen from among ethylene glycol dimethacrylate, trimethylolpropane triacrylate, diallyl phthalate, allyl acrylate, the allyl maleates, methylene-bis-acrylamide, methylene-bis-methacrylamide, tetrallyloxyethane, the triallyl cyanurates, the allylic ethers obtained from polyols.

11. Emulsion according to one of claims 8 to 10, **characterized in that** it has a particle size between 50 nm and 500 nm.

12. Emulsion according to one of claims 8 to 11, **characterized in that** the polymer presents a mean molar mass by weight of between 20,000 g/mol and 1,000,000 g/mol.

13. Emulsion according to one of claims 8 to 12, **characterized in that** it is free of surfactants and solvents other than water.

14. Use of the emulsion according to one of claims 8 to 13, as a thickening agent of an aqueous formulation.

15. Use according to claim 14, **characterized in that** said formulation is selected from an aqueous paint, a paper coating dispersion, an aqueous suspension of mineral substances, a detergent, a cosmetic formulation or containing a hydraulic binder, more specifically, a cosmetic formulation to be applied to the skin or a region of the body where the pH is between 5 and 6.

16. Aqueous formulation containing the emulsion according to one of claims 8 to12, the said formulation being selected from an aqueous paint, a paper coating dispersion, an aqueous suspension of mineral substances, a detergent, a cosmetic formulation, or containing a hydraulic binder, a cosmetic formulation to be applied to the skin or a region of the body where the pH is between 5 and 6.
